# EUROPEAN PATENT APPLICATION

(11) **EP 4 293 114 A1**
(43) Date of publication of application: **20.12.2023**
(21) Application number: 22752813.0
(22) Date of filing: 10.02.2022
(51) Int. Cl.: C12N 15/10, C12Q 1/6806, C12Q 1/6869

(54) **PREPARATION METHOD FOR NUCLEIC ACID SAMPLE DERIVED FROM SKIN STRATUM CORNEUM**

(30) Priority: 12.02.2021 JP 2021020853
(71) Applicant: Kao Corporation, Chuo-ku Tokyo 103-8210 (JP)
(72) Inventor: INOUE, Takayoshi, Haga-gun, Tochigi 321-3497 (JP); YANO, Michiko, Haga-gun, Tochigi 321-3497 (JP)
(74) Representative: Vossius & Partner Patentanwälte Rechtsanwälte mbB
(86) International application number: PCT/JP2022/005355
(87) International publication number: WO 2022/172991

(57) **Abstract**

A method for preparing a nucleic acid sample derived from skin stratum corneum includes: extracting nucleic acid from stratum corneum collected from a skin surface to prepare a water-soluble alcohol aqueous solution containing the nucleic acid; contacting the nucleic acid in the water-soluble alcohol aqueous solution with a solid-phase material to separate the nucleic acid; and amplifying, or reverse-transcribing and amplifying, the separated nucleic acid in the presence of single-stranded nucleic acid-binding protein to prepare a nucleic acid sample.

## Description

### Field of the Invention

The present invention relates to a method for preparing a nucleic acid sample derived from skin stratum corneum.

### Background of the Invention

The skin is tissue covering the outer surface of the body and exposed to the outside environment and is therefore tissue from which a biological sample can be easily and less-invasively collected. Skin tissue can be collected from the body by biopsy, but a method of peeling skin tissue using adhesive tape is also used as a method with less invasiveness. Patent Literature 1 discloses a method of detecting gene expression in the skin by attaching adhesive tape to a target region of the skin to isolate an epidermis (stratum corneum) sample and detecting nucleic acid molecules included in the obtained epidermis sample. However, when skin tissue is collected using adhesive tape, the amount of the stratum corneum sample that can be collected with one strip of tape is small, and the amount of nucleic acid that can be extracted therefrom is further small, which may be insufficient for genetic analysis with high precision. Accordingly, Patent Literature 1 describes that the peeling amount of stratum corneum is increased using tape containing a rubber-based adhesive agent having a stronger adhesive force instead of acrylic adhesive tape that is generally used in peeling of stratum corneum by adhesive tape, and that also a deeper layer of stratum corneum is collected by further attaching the tape to the same skin area multiple times. However, invasiveness is increased with an increase in the depth and area of the stratum corneum to be collected. In Non Patent Literature 1, RNA is extracted from two strips of acrylic adhesive tape with less invasiveness used for collection, and comprehensive analysis of RNA expression is performed. However, the number of gene reads per sample in the report does not satisfy conditions generally required in common analysis, there is a risk that an accurate analysis is not always performed.

In extraction of nucleic acid, such as RNA, from a biological sample, in general, a phenol-chloroform method, an acid guanidinium thiocyanate-phenol-chloroform extraction (AGPC) method, a modified method thereof, or the like is used. Nucleic acid extraction reagents based on these principles are commercially available. Patent Literature 2 describes a nucleic acid extraction method including elution of nucleic acid included in a biological material that has been dissolved, preparation of a lysate solution by adding a water-soluble organic solvent to the solution containing the eluted nucleic acid at a final concentration of from 10 to 60 vol%, and collection of the nucleic acid by contacting the lysate solution with a solid material to allow the nucleic acid to adsorb on the solid material.

Multiplex PCR is a method for simultaneously amplifying multiple regions from one DNA sample by PCR reaction simultaneously using multiple primer pairs. Multiplex RT-PCR, which is a combination of reverse transcription (RT) reaction of RNA to cDNA and multiplex PCR, is used in gene expression analysis, genotyping, and so on.

Non Patent Literature 2 describes that BSA and T4 gene 32 protein (T4GP32) improve PCR reaction in the presence of an inhibitor. Non Patent Literatures 3 and 4 describe that in RT-PCR, the yield of the RT-PCR product was significantly increased by adding T4GP32 to the RT system, but no clear increase in the yield was observed when T4GP32 was added to the PCR system after RT. Patent Literature 3 describes that in a reverse transcription amplification method (RT-RamDA method) for amplifying cDNA using RNA as a template, the yield of cDNA is increased by protecting the cDNA from nuclease decomposition by binding T4GP32 to the single-stranded cDNA peeled from the template RNA. Non Patent Literature 5 describes that DMSO and betaine improve the specificity and yield of PCR.

Patent Literature 1: JP-A-2007-531529
Patent Literature 2: JP-A-2007-117084
Patent Literature 3: WO 2016/052619
Non Patent Literature 1: J. Dermatol. Sci., 2021, 101(1): 14-21
Non Patent Literature 2: Appl. Environ. Microbiol., 1996, 62 (3): 1102-1106
Non Patent Literature 3: Appl. Environ. Microbiol., 1998, 64 (2): 669-677
Non Patent Literature 4: BioTechniques, 2001, 31(1): 81-86
Non Patent Literature 5: PLOS ONE, 2010, 5(6): e11024

### Summary of the Invention

The present invention provides a method for preparing a nucleic acid sample derived from skin stratum corneum, the method comprising:
extracting nucleic acid from stratum corneum collected from a skin surface to prepare a water-soluble alcohol aqueous solution containing the nucleic acid, wherein the stratum corneum is stratum corneum within a depth of 5 **µ**m from the skin surface;
contacting the nucleic acid in the water-soluble alcohol aqueous solution with a solid-phase material to separate the nucleic acid, wherein a concentration of the water-soluble alcohol in the water-soluble alcohol aqueous solution is from 39 to 50 vol% before the contact with the solid-phase material; and
amplifying, or reverse-transcribing and amplifying, the separated nucleic acid in the presence of single-stranded nucleic acid-binding protein to prepare a nucleic acid sample, wherein the amplification is amplification by multiplex PCR, and the single-stranded nucleic acid-binding protein is T4GP32 or a variant thereof.
The present invention provides a method for detecting gene expression in skin, the method including detecting gene expression in a nucleic acid sample derived from skin stratum corneum prepared by the method above.

### Detailed Description of the Invention

All patent literatures, non-patent literatures, and other publications cited herein are hereby incorporated by reference in their entirety.

In the present specification, the phrase "identity of at least 90%" regarding an amino acid sequence or nucleotide sequence refers to an identity of 90% or more, preferably 95% or more, more preferably 97% or more, further more preferably 98% or more, and further more preferably 99% or more.

In the present specification, the identity of a nucleotide sequence or amino acid sequence is calculated by a Lipman-Pearson method (Science, 1985, 227: 1435-41). Specifically, the identity is calculated by analysis using the homology analysis (Search homology) program of genetic information processing software Genetyx-Win (Ver. 5.1.1, Software Development) set the Unit size to compare (ktup) to 2.

The present invention provides a method for preparing a nucleic acid sample derived from skin stratum corneum.

The present inventors found that nucleic acid derived from skin stratum corneum can be prepared with a high yield from stratum corneum of a less invasive shallow layer by extracting nucleic acid from the shallow layer of skin stratum corneum, preparing a solution containing the nucleic acid and a predetermined concentration of a water-soluble alcohol, separating the nucleic acid from the solution, and then subjecting the separated nucleic acid to reverse transcription and/or amplification in the presence of single-stranded nucleic acid-binding protein T4GP32.

According to the present invention, a nucleic acid sample can be prepared with a high yield from a small amount of a biological sample collected from a part suitable for less invasive collection, such as the shallow layer of stratum corneum. The present invention contributes to improvement in the sensitivity, precision, or efficiency of various analyses (e.g., genetic analysis and diagnosis) using a nucleic acid sample derived from a living body.

The nucleic acid prepared by the method of the present invention may be any type of nucleic acid as long as it can be contained in stratum corneum, and examples thereof include RNA, DNA, PNA, and oligonucleotides. The nucleic acid is preferably RNA or DNA. Such nucleic acid may be a single strand or a double strand. Examples of RNA include mRNA, tRNA, rRNA, small RNA (e.g., microRNA (miRNA), small interfering RNA (siRNA), and Piwi-interacting RNA (piRNA)), and long intergenic non-coding (line) RNA and a combination of two or more thereof. Examples of DNA include genomic DNA, mitochondrial DNA, and a combination thereof.

The subject from which stratum corneum is collected may be any organism having skin (epidermis). Examples of the subject include mammals including humans and non-human mammals and are preferably humans. For example, the subject can be a human or a non-human mammal who or which requires or desires analysis of its own nucleic acid. Alternatively, the subject can be a human or a non-human mammal who or which requires or desires gene expression analysis of stratum corneum or other analyses using nucleic acid derived from stratum corneum.

Examples of the part of skin from which stratum corneum of a subject is collected include, but not limited to, skin of any part of the body, such as head, face, neck, trunk, and limbs, healthy skin, skin with a disease such as atopy, acne, dryness, inflammation (redness), and tumor, and skin with a wound.

From the viewpoint of invasiveness to a subject, the stratum corneum to be used for extraction of nucleic acid in the present invention is derived from a shallow layer of stratum corneum and is specifically stratum corneum within a depth of 5 **µ**m from the skin surface. In the present invention, stratum corneum within a depth of 5 **µ**m from the skin surface is collected, and nucleic acid contained therein is extracted. In the present invention, preferably, stratum corneum within a depth of 2.6 **µ**m from the skin surface is collected, and nucleic acid contained therein is extracted. Stratum corneum in such a region contains many RNAs related to keratinization and synthesis or metabolism of lipid (Examples described later, Table 4). In the present invention, preferably, stratum corneum in a layer deeper than 5 **µ**m, more preferably 2.6 **µ**m, from the skin surface is not collected. A depth of within 5 **µ**m from the skin surface corresponds to a thickness of three or four stratum corneum layers, and a depth of 2.6 **µ**m from the skin surface corresponds to a thickness of one or two stratum corneum layers.

In the collection of skin stratum corneum from a subject, any means that can collect mainly a shallow layer of stratum corneum can be adopted, and examples thereof include a method of attaching a glass plate, adhesive tape, or the like to a skin surface and allowing the stratum corneum of a shallow layer to adhere thereto, and a method of scraping and collecting stratum corneum from a skin surface with a spatula, scraper, or the like. Among these methods, preferred is the method using adhesive tape (so-called tape stripping). The adhesive tape is composed of an adhesive layer with an adhesive force and a support for holding the layer. The shape and material of the support are not particularly limited as long as it is possible to hold the adhesive layer and the adhesive agent of the adhesive layer is not affected. The support preferably has flexibility suitable for closely sticking to a skin surface and is inert to extraction of nucleic acid from stratum corneum described later. The adhesive agent constituting the adhesive layer is not particularly limited as long as it has an adhesive force allowing adhesion and peeling of stratum corneum, and examples thereof include acrylic adhesive agent, rubber-based adhesive agent, silicone-based adhesive agent, and urethane-based adhesive agent. In the present invention, preferably used is an adhesive agent having an adhesive force that can peel stratum corneum from the skin surface up to a depth of 5 **µ**m by tape stripping from once to several times, and for example, acrylic adhesive agents are preferable. The size of the adhesive tape is not particularly limited, but is, for example, within a range of from 0.5 to 25 cm², preferably within a range of from 1 to 17.5 cm², and more preferably within a range of from 2 to 10 cm². Examples of such adhesive tape include disk-like acrylic adhesive tape, D-Squame (diameter: 2.2 cm, Clinical & Derm). Before collection of stratum corneum by the procedure above, it is preferable to remove sebum, stain, and so on from the target region of the skin from which stratum corneum is collected. In a preferable embodiment of the tape stripping procedure, tape may be attached to the target region of skin such that the adhesive surface is in contact with the skin surface, the tape may be pressed to the skin such that a uniform force, for example, a uniform force within a range of from 100 to 300 g/cm², preferably within a range of from 200 to 250 g/cm², is applied across the tape, and then the tape may be removed with a uniform force.

Stratum corneum can be collected repeatedly from the same target region of skin. For example, in the case of tape stripping, the method of the present invention includes collection of stratum corneum by attaching adhesive tape to a target region of skin once or to the same target region multiple times to allow the stratum corneum to adhere to the tape. Stratum corneum of a deeper layer can be collected depending on the number of times the tape is attached to the skin. The number of times of attaching tape can be appropriately changed depending on the adhesive force of the tape such that stratum corneum at a desired depth can be collected. Although the method of the present invention does not necessarily exclude attachment of the same tape to skin multiple times, preferably, multiple strips of tape are respectively attached to skin once. In the method of the present invention, one or two strips of adhesive tape are preferably attached to the same target region of skin, and each strip of the tape is preferably attached to the skin once. Consequently, stratum corneum within a depth of 5 **µ**m, preferably 2.6 **µ**m, from the skin surface adheres to the tape and is collected.

The nucleic acid included in the collected stratum corneum can be extracted in accordance with a common method. For example, in the case of tape stripping, nucleic acid can be extracted from the stratum corneum adhered to the tape by, for example, a phenol-chloroform method, a phenol-chloroform-isoamyl alcohol (PCI) method, an acid guanidinium thiocyanate-phenol-chloroform extraction (AGPC) method, or a modified method thereof, a method of using special magnetic particles coated with silica, a method of using solid phase reversible immobilization magnetic particles, or using a commercially available nucleic acid purification reagent (e.g., TRIzol (Registered Trademark) Reagent, RNeasy (Registered Trademark), QIAzol (Registered Trademark), ISOGEN, DNeasy, DNAzol, and ISOGENOME).

The extracted nucleic acid is preferably prepared as an aqueous solution containing the nucleic acid. For example, in the phenol-chloroform method, a water layer (upper layer) containing the nucleic acid and a phenol-chloroform layer (lower layer) can be separated from each other. Accordingly, an aqueous solution containing the nucleic acid can be prepared by collecting the water layer.

As an embodiment, an example of the RNA extraction procedure by a phenol-chloroform method will now be described. Acidic phenol is added to and mixed with a sample including nucleic acid (e.g., a solution containing dissolved stratum corneum adhered to the above-described adhesive tape), and chloroform is then added thereto and mixed therewith to extract RNA from the sample. Subsequently, the obtained mixture solution is centrifuged and separated into a water layer (upper layer) containing RNA and a phenol-chloroform layer (lower layer) (and an intermediate layer may be also separated in some cases). The water layer is collected to prepare an aqueous solution containing RNA. The acidic phenol to be used in RNA extraction may be water saturated phenol alone or may be a reagent mixture solution containing phenol. For example, a reagent solution for extracting RNA from a biological sample, such as a commercially available RNA extraction reagent containing guanidine thiocyanate (e.g., TRIzol (Registered Trademark) Reagent, QIAzol Lysis Reagent, Isogen Life Science B.V.), can be used as the reagent mixture solution containing phenol. From the viewpoint of the efficiency of RNA extraction from a sample, it is preferable to use a commercially available reagent solution for RNA extraction as described above. That is, RNA extraction is preferably performed by a modified AGPC method. As the chloroform to be used for the RNA extraction, chloroform is preferably used alone, but a reagent mixture solution containing chloroform may be used as long as a necessary amount of RNA is distributed in the water layer. As needed, the prepared aqueous solution containing RNA may be mixed with phenol and chloroform again, and the water layer may be collected by centrifugation. This procedure may be repeated twice or more. Furthermore, the prepared aqueous solution containing the RNA may be mixed with chloroform again, and the water layer may be collected by centrifugation. This procedure may be repeated twice or more.

In the present invention, a water-soluble alcohol aqueous solution containing the extracted nucleic acid (hereinafter, also referred to as a nucleic acid-containing water-soluble alcohol aqueous solution) is prepared. For example, the extracted nucleic acid or an aqueous solution containing it and a water-soluble alcohol or a water-soluble alcohol aqueous solution may be mixed. Examples of the water-soluble alcohol include primary alcohol, secondary alcohol, and tertiary alcohol. Among them, methanol, ethanol, isopropanol, n-propanol, and butanol can be preferably used. Butanol may be linear or branched. These water-soluble alcohols can be used alone or in combination of two or more thereof. From the viewpoint of reducing the environmental load and toxicity to workers, ethanol is more preferable. The final concentration of the water-soluble alcohol in the nucleic acid-containing water-soluble alcohol aqueous solution is, at the time before the contact with a solid-phase material, from 39 to 50 vol%, preferably from 40 to 46 vol%, more preferably from 41 to 45.5 vol%, and further more preferably from 42 to 45 vol% from the viewpoint of improving the yield of nucleic acid in the separation of nucleic acid using a solid-phase material described later. The nucleic acid-containing water-soluble alcohol aqueous solution is preferably prepared such that the final concentration of the water-soluble alcohol is from 39 to 50 vol%, preferably from 40 to 46 vol%, more preferably from 41 to 45.5 vol%, and further more preferably from 42 to 45 vol%, and this aqueous solution is contacted with a solid-phase material. In the present invention, the vol% means vol% at 25°C and 1 atm.

Subsequently, nucleic acid is separated from the nucleic acid-containing water-soluble alcohol aqueous solution. The separation of nucleic acid from the nucleic acid-containing water-soluble alcohol aqueous solution can be performed in accordance with a nucleic acid separation method using a solid-phase material. For example, the nucleic acid-containing water-soluble alcohol aqueous solution containing a water-soluble alcohol at the final concentration above is contacted with a solid-phase material to adsorb the nucleic acid in the solution to the solid-phase material. Subsequently, contaminants are washed away from the solid-phase material as needed, and the nucleic acid is then desorbed from the solid-phase material and is collected.

The solid-phase material may be nucleic acid-adsorbing solid-phase material, and examples thereof include silica-based solid-phase material. From the viewpoint of the adsorption of nucleic acid, the solid-phase material is preferably a solid-phase material including a porous silica membrane. The solid-phase material is preferably in the form of a spin column, a pipette tube with a column, or a column cartridge that can be attached to a centrifugal tube or a pipette tube from the viewpoint of making the operations of washing away of contaminants and elution of nucleic acid easy, and is more preferably in the form of a spin column or a column cartridge that can be attached to a centrifugal tube from the viewpoint also of contamination prevention and operation efficiency. Furthermore, considering the relatively small amount of nucleic acid to be collected, the solid-phase material is preferably in the form that can purify a small amount of nucleic acid, such as a miniprep column. As the solid-phase material, a commercially available column for purifying DNA or RNA can be used, and examples thereof include RNeasy (Registered Trademark) Spin Column (QIAGEN N.V.), QIAamp Mini Spin Column (QIAGEN N.V.), and NucleoSpin (Registered Trademark) RNA Column (Takara Bio Inc.).

As the cleaning liquid that is used in washing away contaminants from the solid-phase material, a solution (e.g., aqueous solution) containing at least one selected from a water-soluble organic solvent and water-soluble salt can be used. As the water-soluble organic solvent included in the cleaning liquid, water-soluble alcohol can be used. Examples of the water-soluble alcohol include methanol, ethanol, propanol, and butanol. Propanol may be isopropanol or n-propanol, and butanol may be linear or branched. These water-soluble alcohols can also be used in combination of two or more thereof. Among them, it is preferable to use ethanol. The amount of the water-soluble organic solvent contained in the cleaning liquid may be an amount that can hold nucleic acid on the solid-phase material and wash away contaminants and the amount can be appropriately determined by those skilled in the art, and is preferably from 30 to 100 vol% and more preferably from 35 to 50 vol%. The water-soluble salt contained in the cleaning liquid is preferably a halide salt, among them, preferably a chloride salt. The water-soluble salt is preferably a monovalent or divalent cation salt, more preferably an alkali metal salt or an alkaline earth metal salt, further more preferably a sodium salt or a potassium salt, and even more preferably a sodium salt. When the water-soluble salt is contained in a cleaning liquid, the concentration thereof is preferably 10 mmol/L or more and more preferably 20 mmol/L or more. The upper limit of the concentration is not particularly limited as long as it does not impair the solubility of impurities, but is preferably 1 mol/L or less and more preferably 0.1 mol/L or less. The water-soluble salt is further more preferably sodium chloride, and the concentration thereof in a cleaning liquid is preferably 20 mmol/L or more and 0.1 mol/L or less.

Desorption of nucleic acid from the solid-phase material can be preferably performed by passing an eluent through the solid-phase material to thereby eluting the nucleic acid. As the eluent to be used for the elution of nucleic acid, water, Tris-EDTA (TE) buffer, or the like can be used. As the cleaning liquid and eluent, the cleaning liquid and eluate included in a commercially available column for nucleic acid purification may be used. Washing of the solid-phase material and elution can be performed by a usual procedure. For example, when the solid-phase material is loaded on a spin column, a nucleic acid-containing alcohol aqueous solution is passed through the column, a cleaning liquid is added to the column on which the nucleic acid is adsorbed, and centrifugation is performed to wash away contaminants together with the cleaning liquid. Subsequently, an eluent is added to the column, and the nucleic acid is eluted from the column by centrifugation.

When the nucleic acid is RNA, the solid-phase material adsorbing RNA may be treated with DNase, for example, RNase-Free DNase set (QIAGEN N.V.) as needed. The DNase treatment removes contaminated DNA and can improve the purity of the collected RNA. The DNase treatment is preferably performed between washing of the solid-phase material and elution of RNA.

Subsequently, in the present invention, the separated nucleic acid is subjected to reverse transcription and/or amplification in the presence of single-stranded nucleic acid-binding protein to prepare a nucleic acid sample. Nucleic acid is amplified by multiplex PCR (MPCR). When the nucleic acid is RNA, the RNA is preferably subjected to reverse transcription (RT) to synthesize cDNA, and the cDNA is then amplified.

Accordingly, when the nucleic acid is DNA, the DNA is amplified by multiplex PCR in the presence of single-stranded nucleic acid-binding protein to prepare a nucleic acid sample. When the nucleic acid is RNA, the RNA is subjected to reverse transcription and amplification by multiplex RT-PCR (RT-MPCR) in the presence of single-stranded nucleic acid-binding protein to prepare a nucleic acid sample.

The reverse transcription (RT) of RNA can be performed by a usual procedure except that single-stranded nucleic acid-binding protein is added to the reaction system. For example, cDNA may be synthesized by incubating a reaction solution containing target RNA to be reverse-transcribed, a primer, a reverse transcriptase, and a substrate in the presence of single-stranded nucleic acid-binding protein to allow the reverse transcriptase to act on the target RNA. As the primer, a primer targeting specific RNA to be analyzed may be used, but it is preferable to use an oligo dT primer, random primer, or a mixture thereof for more comprehensive preservation and analysis of nucleic acid. As the reverse transcriptase, a general reverse transcriptase can be used, but it is preferable to use a reverse transcriptase showing high accuracy and efficiency of RT (for example, M-MLV Reverse Transcriptase or a variant thereof, or a commercially available enzyme for reverse transcription described later). The substrate is a substrate for the reverse transcriptase, and a deoxyribonucleotide, e.g., dATP, dCTP, dGTP, dTTP, and a mixture thereof, can be preferably used. The deoxyribonucleotide may be modified. In the present invention, examples of the enzyme and other reagents for RT include commercially available enzymes for reverse transcription and reverse transcription reagent kits, for example, PrimeScript (Registered Trademark) Reverse Transcriptase series (Takara Bio Inc.) and Superscript (Registered Trademark) Reverse Transcriptase series (Life Technologies Japan Ltd.), and Superscript (Registered Trademark) III Reverse Transcriptase and Superscript (Registered Trademark) VILO cDNA Synthesis kit (both available from Life Technologies Japan Ltd.) can be preferably used. The temperature and time of the RT reaction can be appropriately set depending on the type of enzyme or reagent to be used, target RNA, cDNA to be synthesized, and so on. For example, a reaction solution is prepared using a commercially available reagent for reverse transcription in accordance with the manual thereof, and single-stranded nucleic acid-binding protein may be added to the reaction solution. The conditions of RT may also be set in accordance with the manual of the reagent.

Amplification of nucleic acid by MPCR can be performed by a usual procedure except that single-stranded nucleic acid-binding protein is added to the reaction system. For example, a reaction solution containing target DNA or cDNA, a plurality of primer pairs, a DNA synthetase, and a substrate may be applied to MPCR in the presence of single-stranded nucleic acid-binding protein. The primer pair can be appropriately designed depending on the target DNA sequence. As the DNA synthetase, a general DNA synthetase can be used, but it is preferable to use a DNA synthetase showing high accuracy and efficiency of amplification. The substrate is a substrate for the DNA synthetase, and a deoxyribonucleotide, e.g., dATP, dCTP, dGTP, dTTP, and a mixture thereof, can be used. The deoxyribonucleotide may be modified. As the enzyme and other reagents for MPCR, commercially available enzymes and reagents can be used. Examples of the enzyme and other reagent for MPCR include Ion AmpliSeq Transcriptome Human Gene Expression Kit (Life Technologies Japan Ltd.). The temperature and time for the amplification reaction can be appropriately set depending on the means of amplification, the type of enzyme or reagent to be used, template DNA, and so on. For example, a reaction solution is prepared using a commercially available reagent for MPCR in accordance with the manual thereof, and single-stranded nucleic acid-binding protein may be added to the reaction solution. The conditions of PCR may also be set in accordance with the manual of the reagent.

Examples of the single-stranded nucleic acid-binding protein that is used for RT and MPCR of nucleic acid include T4GP32 (T4 gene 32 protein) and variants thereof. T4GP32 is protein typically consisting of the amino acid sequence of SEQ ID NO: 1 and binds to single-stranded DNA. Examples of the variant of T4GP32 include protein obtained by modification or mutation of an arbitrary amino acid on the amino acid sequence of T4GP32. Examples of the variant of T4GP32 include protein that consists of an amino acid sequence having a sequence identity of at least 90% with the amino acid sequence of SEQ ID NO: 1 and binds to single-stranded nucleic acid, preferably single-stranded DNA. T4GP32 can be purchased (for example, from NEW ENGLAND BioLabs, Inc. or Sigma-Aldrich). The initial concentration of the single-stranded nucleic acid-binding protein in the reaction solution for RT or MPCR (the final concentration in the solution before reaction) may be preferably 3 **µ**g/mL or more, more preferably 9 **µ**g/mL or more, and further more preferably 20 **µ**g/mL or more and preferably 300 **µ**g/mL or less, more preferably 200 **µ**g/mL or less, and further more preferably 150 **µ**g/mL or less, or preferably from 3 to 300 **µ**g/mL, more preferably from 9 to 200 **µ**g/mL, and further more preferably from 20 to 150 **µ**g/mL.

In the method of the present invention, RT and MPCR of RNA are preferably performed in different reaction systems (2 steps), but may be performed in one reaction system (1 step) in terms of simplicity. In the case of 1 step reaction, the single-stranded nucleic acid-binding protein used for RT can be used for MPCR as it is. For example, the initial concentration of single-stranded nucleic acid-binding protein in the reaction solution for RT-MPCR may be adjusted to preferably 3 **µ**g/mL or more, more preferably 9 **µ**g/mL or more, and further more preferably 20 **µ**g/mL or more, and may be adjusted to preferably 300 **µ**g/mL or less, more preferably 200 **µ**g/mL or less, and further more preferably 150 **µ**g/mL or less, or may be adjusted to preferably from 3 to 300 **µ**g/mL, more preferably from 9 to 200 **µ**g/mL, and further more preferably from 20 to 150 **µ**g/mL.

The reaction product obtained by the reverse transcription and/or MPCR above can be utilized as a nucleic acid sample derived from skin stratum corneum. The reaction product may be directly used as a nucleic acid sample or is purified as needed to be used as a nucleic acid sample.

Purification of the nucleic acid amplified by reverse transcription and/or MPCR is preferably performed by size separation. The size separation can separate the amplification product (the target nucleic acid) from the primers and other impurities contained in the reaction liquid. The size separation of nucleic acid can be performed with, for example, a size separation column, a size separation chip, or magnetic beads that can be used for size separation. Preferred examples of the magnetic beads that can be used for size separation include solid phase reversible immobilization (SPRI) magnetic beads such as Ampure XP. For example, when Ampure XP and a DNA solution are mixed, DNA is adsorbed to the carboxyl group coated on the surface of the magnetic beads, and only the magnetic beads are collected using magnet to purify the DNA. The molecular size of DNA adsorbing to the magnetic beads changes by changing the mixing ratio of the Ampure XP solution and the DNA solution. DNA having a specific molecular size desired to be captured is collected on the magnetic beads by utilizing this principle, and DNA with another molecular size and impurities can be purified.

The purified nucleic acid may be further subjected to treatment required for subsequent analysis. For example, if the purified nucleic acid is cDNA obtained by reverse transcription, amplification can be performed by the procedure above. For example, for sequencing or fragment analysis of DNA, the purified DNA may be prepared into an appropriate buffer solution, the primer region included in the DNA may be cut, or an adaptor sequence may be further added to the DNA. For example, a library for various analyses can be prepared by preparing the purified DNA into a buffer solution, subjecting the DNA to removal of a primer sequence and adaptor ligation, and amplifying the obtained reaction product as needed. These operations can be performed, for example, using 5×VILO RT Reaction Mix included in Superscript (Registered Trademark) VILO cDNA Synthesis kit (Life Technologies Japan Ltd.) and 5×Ion AmpliSeq HiFi Mix and Ion AmpliSeq Transcriptome Human Gene Expression Core Panel included in Ion AmpliSeq Transcriptome Human Gene Expression Kit (Life Technologies Japan Ltd.) in accordance with the protocol attached to each kit.

The nucleic acid sample prepared in the present invention can be used in various analyses or diagnoses using nucleic acid, for example, comprehensive analysis of gene or expression analysis targeting a specific gene, such as gene expression analysis, transcriptome analysis, functional analysis of a subject, diagnosis of a disease, or efficacy evaluation of a drug administered to a subject. Accordingly, the present invention also relates to use, for various analysis, of a nucleic acid sample derived from skin stratum corneum, the sample being prepared by the above-described method for preparing a nucleic acid sample by the present invention. In one embodiment, the present invention provides a method for detecting gene expression in skin, the method including detecting gene expression in a nucleic acid sample derived from skin stratum corneum prepared by the above-described method for preparing a nucleic acid sample by the present invention.

In the method for detecting gene expression by the present invention, as long as the nucleic acid sample derived from skin stratum corneum prepared by the method for preparing a nucleic acid sample by the present invention is used, the procedure and means for detecting gene expression are not particularly limited. For example, when gene expression is detected by sequencing, a library is prepared by adaptor ligation of amplified and purified nucleic acid, and the library can be applied to sequencing. It is possible to detect or quantitatively measure gene expression based on the number of reads of the sequence derived from each gene obtained by the sequencing.

As exemplary embodiments of the present invention, the following materials, manufacturing methods, uses, methods, and so on are further disclosed herein. However, the present invention is not limited to these embodiments.

[1] A method for preparing a nucleic acid sample derived from skin stratum corneum, the method comprising:
   extracting nucleic acid from stratum corneum collected from a skin surface to prepare a water-soluble alcohol aqueous solution containing the nucleic acid, wherein the stratum corneum is stratum corneum within a depth of 5 **µ**m from the skin surface;
   contacting the nucleic acid in the water-soluble alcohol aqueous solution with a solid-phase material to separate the nucleic acid, wherein a concentration of the water-soluble alcohol in the water-soluble alcohol aqueous solution is from 39 to 50 vol% before the contact with the solid-phase material; and
   amplifying, or reverse-transcribing and amplifying, the separated nucleic acid in the presence of single-stranded nucleic acid-binding protein to prepare a nucleic acid sample, wherein the amplification is amplification by multiplex PCR, and the single-stranded nucleic acid-binding protein is T4GP32 or a variant thereof.
[2] The method according to [1], preferably further comprising collecting stratum corneum from the skin surface.
[3] The method according to [1] or [2], wherein the stratum corneum collected from the skin surface is:
   preferably stratum corneum within four stratum corneum layers from the skin surface and more preferably stratum corneum within two stratum corneum layers from the skin surface; or
   preferably stratum corneum within a depth of 2.6 **µ**m from the skin surface and not including stratum corneum in a layer deeper than 5 **µ**m from the skin surface.
[4] The method according to any one of [1] to [3], wherein the collection of stratum corneum from a skin surface is preferably collection of stratum corneum by tape stripping.
[5] The method according to [4], wherein adhesive tape used in the tape stripping is preferably acrylic adhesive tape.
[6] The method according to [5], wherein the collection of stratum corneum by tape stripping preferably includes attaching one or two strips of the acrylic adhesive adhesive tape to a target region of skin to allow stratum corneum to adhere to the tape and peeling the stratum corneum.
[7] The method according to any one of [1] to [6], wherein the nucleic acid extracted from stratum corneum is preferably RNA or DNA.
[8] The method according to [7], wherein the nucleic acid extracted from stratum corneum and the separated nucleic acid are preferably RNA, and the separated RNA is subjected to reverse transcription and amplification in the presence of the single-stranded nucleic acid-binding protein to prepare a nucleic acid sample.
[9] The method according to [8], wherein an initial concentration of the single-stranded nucleic acid-binding protein in a reaction solution for the reverse transcription and amplification is:
   preferably 3 **µ**g/mL or more, more preferably 9 **µ**g/mL or more, further more preferably 20 **µ**g/mL or more and preferably 300 **µ**g/mL or less, more preferably 200 **µ**g/mL or less, and further more preferably 150 **µ**g/mL or less; or
   preferably from 3 to 300 **µ**g/mL, more preferably from 9 to 200 **µ**g/mL, and further more preferably from 20 to 150 **µ**g/mL.
[10] The method according to [9], wherein the reverse transcription and amplification are preferably multiplex RT-PCR.
[11] The method according to [7], wherein the nucleic acid extracted from stratum corneum and the separated nucleic acid are preferably DNA, and the separated DNA is amplified in the presence of the single-stranded nucleic acid-binding protein to prepare a nucleic acid sample.
[12] The method according to [11], wherein an initial concentration of the single-stranded nucleic acid-binding protein in a reaction solution for the amplification is:
   preferably 3 **µ**g/mL or more, more preferably 9 **µ**g/mL or more, further more preferably 20 **µ**g/mL or more and preferably 300 **µ**g/mL or less, more preferably 200 **µ**g/mL or less, and further more preferably 150 **µ**g/mL or less; or
   preferably from 3 to 300 **µ**g/mL, more preferably from 9 to 200 **µ**g/mL, and further more preferably from 20 to 150 **µ**g/mL.
[13] The method according to any one of [1] to [12], wherein the water-soluble alcohol is:
   preferably at least one selected from the group consisting of methanol, ethanol, isopropanol, n-propanol, and butanol; and
   more preferably ethanol.
[14] The method according to any one of [1] to [13], wherein the T4GP32 or a variant thereof is preferably protein that consists of an amino acid sequence of SEQ ID NO: 1 or an amino acid sequence having a sequence identity of at least 90% with the sequence and binds to single-stranded nucleic acid.
[15] The method according to any one of [1] to [14], wherein the nucleic acid is extracted from stratum corneum preferably by a phenol-chloroform method or a modified method thereof.
[16] The method according to any one of [1] to [15], wherein a concentration of the water-soluble alcohol in the water-soluble alcohol aqueous solution is preferably from 40 to 46 vol%, more preferably from 41 to 45.5 vol%, and further more preferably from 42 to 45 vol%.
[17] The method according to any one of [1] to [16], wherein the solid-phase material is:
   preferably a silica-based solid-phase material; and
   more preferably a solid-phase material including a porous silica membrane.
[18] The method according to [17], wherein separation of nucleic acid in the water-soluble alcohol aqueous solution includes allowing nucleic acid in the water-soluble alcohol aqueous solution to adsorb to the silica-based solid-phase material.
[19] The method according to [18], wherein the solid-phase material is preferably in the form of a spin column, a pipette tube with a column, or a column cartridge that can be attached to a centrifugal tube or pipette tube.
[20] The method according to [18] or [19], preferably further comprising collecting nucleic acid adsorbed to the solid-phase material.
[21] The method according to [20], preferably comprising washing the solid-phase material to which nucleic acid adsorbed and then eluting and collecting the nucleic acid from the solid-phase material.
[22] The method according to [21], wherein
   the cleaning liquid for washing the solid-phase material is preferably a solution including at least one selected from the group consisting of a water-soluble organic solvent and a water-soluble salt, wherein
   the water-soluble organic solvent is preferably water-soluble alcohol, more preferably at least one selected from the group consisting of methanol, ethanol, isopropanol, n-propanol, and butanol, and further more preferably ethanol, and
   the water-soluble salt is preferably a monovalent or divalent cation salt, more preferably an alkali metal salt or an alkaline earth metal salt, and further more preferably a sodium salt or a potassium salt, and preferably a chloride salt, and even preferably sodium chloride.
[23] The method according to [22], wherein a concentration of the water-soluble organic solvent in the cleaning liquid is preferably from 30 to 100 vol% and more preferably from 35 to 50 vol%.
[24] The method according to [22], wherein a concentration of the water-soluble salt in the cleaning liquid is preferably 10 mmol/L or more and 1 mol/L or less, more preferably 10 mmol/L or more and 0.1 mol/L, further more preferably 20 mmol/L or more and 1 mol/L or less, and further more preferably 20 mmol/L or more and 0.1 mol/L or less.
[25] The method according to any one of [21] to [24], wherein an eluent for eluting nucleic acid from the solid-phase material is preferably water or a buffer.
[26] The method according to any one of [1] to [25], wherein the reverse transcription is performed:
   preferably in a reaction solution containing an oligo dT primer, a random primer, or a mixture thereof; and
   more preferably in a reaction solution including a random primer.
[27] A method for detecting gene expression in skin, the method comprising preferably detecting gene expression in a nucleic acid sample derived from skin stratum corneum prepared by the method according to any one of [1] to [26] .
[28] The method according to [27], wherein the gene expression is preferably detected by sequencing.

### Examples

The present invention will now be described in more detail based on examples, but is not limited thereto.

### Test 1: Detection of gene expression in skin stratum corneum: comparison by library preparation method 1) Collection of stratum corneum and RNA extraction

Subjects were two healthy males and females (four in total) in their 30s to 50s. Stratum corneum was collected from two areas, both the left and right face cheeks, of each subject using one strip of D-Squame tape (disk-like shape with a diameter of 2.2 cm, manufactured by Clinical & Derm). Specifically, the tape was attached to the sampling site of each cheek of the subjects after face washing, a constant pressure of about 225 g/cm² was applied to the tape, and the tape was then slowly removed at a constant speed to collect stratum corneum. One milliliter of QIAzol Lysis Reagent (QIAGEN N.V.) was added to each tape collected stratum corneum to dissolve the stratum corneum adhered to the tape surface, and the stratum corneum was collected in a tube. Two hundred microliters of chloroform were added thereto, followed by mixing and centrifugation (15,000 rpm, 15 min, 4°C). The water layer (upper layer) was collected in a new tube as a sample. Two samples prepared from one subject were respectively treated in accordance with different methods (method A and method B below) to produce libraries for gene expression detection.

### 2) Preparation of library for gene expression detection Method A)

One of two samples prepared in 1) was mixed with an equal volume of 70% (v/v) ethanol, and RNA was purified from the ethanol aqueous solution containing the obtained sample using a silica-based column (RNeasy mini kit, QIAGEN N.V.) in accordance with the protocol of the kit.

From the purified RNA, cDNA was synthesized by reverse transcription at 42°C for 90 minutes using Superscript (Registered Trademark) VILO cDNA Synthesis kit (Life Technologies Japan Ltd.).

A library containing DNA derived from 20,802 genes that can be detected by multiplex PCR was prepared from the obtained cDNA. The multiplex PCR was performed using Ion AmpliSeq Transcriptome Human Gene Expression Kit (Life Technologies Japan Ltd.) under conditions of [99°C, 2 min -> 20 cycles of (99°C, 15 sec -> 62°C, 16 min) -> 4°C for hold].

The obtained PCR products were purified with Ampure XP (Beckman Coulter, Inc.), and then buffer reconstitution, primer sequence digestion, adaptor ligation, purification, and amplification were performed to prepare a library.

The yields of the RNA, PCR products, and library prepared by this procedure were measured using Agilent 4200 TapeStation system (Agilent technologies).

### Method B)

The other of two samples prepared in 1) was mixed with an equal volume of 85% (v/v) ethanol, and RNA was purified from the ethanol aqueous solution containing the obtained sample using a silica-based column (RNeasy mini kit, QIAGEN N.V.) in accordance with the protocol of the kit.

From the purified RNA, cDNA was synthesized by reverse transcription at 42°C for 90 minutes using Superscript (Registered Trademark) VILO cDNA Synthesis kit (Life Technologies Japan Ltd.). In the reverse transcription, T4GP32 (NEW ENGLAND BioLabs, Inc.) was added to the reaction solution at a final concentration of 100 **µ**g/mL.

A library containing DNA derived from 20,802 genes that can be detected by multiplex PCR was prepared from the obtained cDNA. The multiplex PCR was performed using Ion AmpliSeq Transcriptome Human Gene Expression Kit (Life Technologies Japan Ltd.) under conditions of [99°C, 2 min -> 20 cycles of (99°C, 15 sec -> 62°C, 16 min) -> 4°C for hold]. In the PCR, T4GP32 (NEW ENGLAND BioLabs, Inc.) was added to the reaction solution at a final concentration of 35 **µ**g/mL.

The obtained PCR products were purified with Ampure XP (Beckman Coulter, Inc.), and then buffer reconstitution, primer sequence digestion, adaptor ligation, purification, and amplification were performed to prepare a library.

The yields of the RNA, PCR products, and library prepared by this procedure were measured using Agilent 4200 TapeStation system (Agilent technologies).

### 3) Gene expression detection

The libraries prepared by the methods A and B were each loaded on Ion 540 Chip, and sequencing was performed using Ion S5/XL system (Life Technologies Japan Ltd.). Each of the read sequences obtained by sequencing was genetically mapped using a reference sequence, hg19 AmpliSeq Transcriptome ERCC v1, of the human genome to determine the gene from which each read sequence was derived. The data (read count values) of the expression level of the measured each gene was corrected using a means called DESeq2. The corrected count value (normalized count value) was used in the detection of gene expression.

### 4) Library yield and comparison of detection gene

The yields of PCR products obtained by multiplex PCR in the methods A and B and the yields of the finally obtained libraries are shown in Table 1 for each subject. The yields of both the PCR product and the library were increased in the method B than those in the method A.

Regarding the libraries prepared by the methods A and B, the genes detected in 3) were compared. Genes showed that the corrected count value was a value of 0 in all the subjects in the library prepared by the method A and the corrected count value was a value of exceeding 0 in at least three subjects in the four subjects in the library prepared by the method B were chosen, and 139 genes were identified (Table 2). In contrast, genes showed that the corrected count value was a value of 0 in all the subjects in the library prepared by the method B and the corrected count value was a value of exceeding 0 in at least three subjects in the four subjects in the library prepared by the method A were chosen, and only 22 genes were identified (data are not shown). This result demonstrated that in the library prepared by the method B, a larger number of genes can be detected, compared to the method A. Furthermore, it was revealed that in the 139 genes (Table 2), genes important for the function of skin, such as TSLP (Cell, 155: 285-295, 2013) deeply involved in itching of skin and MC1R (Cancer Manag. Res., 10: 1143-1154, 2018) deeply involved in melanoma, are included.

**[Table 1]**

| Subject | Multiplex PCR yield | | | Library yield | |
|---|---|---|---|---|---|
| | Method A (pg/µL) | Method B (pg/µL) | | Method A (pg/µL) | Method B (pg/µL) |
| Male 1 | 399 | 404 | | 513 | 546 |
| Male 2 | 141 | 544 | | 246 | 726 |
| Female 1 | 323 | 6060 | | 609 | 7000 |
| Female 2 | 1600 | 8580 | | 2770 | 7450 |
| Average | 615.75 | 3897 | | 1034.5 | 3930.5 |

**[Table 2]**

| Gene groups detected only in method B | | | | |
|---|---|---|---|---|
| ABCD1 | CLIP2 | IRF4 | POLG2 | TUBB3 |
| ACCS | CNIH3 | ITGAM | POU2F2 | UHRF1 |
| ACSM3 | COQ10A | KIF5A | PTBP2 | ZBED2 |
| ADCK1 | CYP4V2 | KNTC1 | PTPRN2 | ZBTB10 |
| ADRA2A | DAPK2 | LARP1B | RASAL3 | ZBTB16 |
| AGER | DARS2 | LGI3 | RAVER1 | ZFHX3 |
| ALDH6A1 | DCAF17 | LHX3 | REC8 | ZNF438 |
| ALG9 | DDX10 | LINC00467 | RNF125 | ZNF462 |
| ANO2 | DLG4 | LOC100506123 | SLC25A35 | ZNF789 |
| ARHGDIA | ERVMER34-1 | LOC100506497 | SLC29A2 | LINC00520 |
| ASB9 | FABP7 | LOC440925 | SLC39A13 | ARL17B |
| ATP6V1G2 | FAM118A | LOC641515 | SLCO4C1 | HYMAI |
| BMP1 | FAM175A | LOC653712 | SMC6 | KIF1A |
| C12orf73 | FAM185A | LOC728819 | SNED1 | LOC100129794 |
| C19orf57 | FLJ40292 | LOXL1 | SNORA55 | LOC100335030 |
| CCDC92 | FLRT2 | MED9 | SNX24 | MC1R |
| CDC20 | GALNT4 | MGC12916 | SOD3 | MRPL42P5 |
| CDC6 | GAS2L3 | MRM1 | SPAG8 | MRPL45P2 |
| CDCA2 | GHDC | MTERFD1 | SPRED3 | ZN F763 |
| CDK1 | GLDN | MTRNR2L4 | STARD8 | |
| CDK20 | GLIPR2 | NKTR | STARD9 | |
| CDRT1 | GLT25D2 | OR1 0A5 | STEAP1 | |
| CECR6 | GLUD2 | OSBPL6 | SULT1A1 | |
| CFB | HAUS8 | PAOX | SYDE2 | |
| CGREF1 | HIST1H2AG | PAQR5 | SYNE1 | |
| CHCHD1 | HIST1H2AJ | PATZ1 | TMEM185B | |
| CHML | HIST1H2BF | PCDHB7 | TMEM97 | |
| CHRM4 | HIST1H2BM | PLA2G16 | TNFRSF10A | |
| CKS2 | IL15RA | PLCG2 | TSHZ3 | |
| CLEC14A | INVS | PLEKHH2 | TSLP | |

### Example 2: Comparison of gene expression profile across stratum corneum depth

### 1) RNA extraction from stratum corneum and RNA sequencing

In a face cheek of a healthy female in her 30s, stratum corneum was collected from the same area up to the depth of 10 strips of tape successively using 10 strips of D-Squame tape (disk-like shape with a diameter of 2.2 cm, manufactured by Clinical & Derm). Specifically, the tape was attached to the sampling site of the cheek of the subject after face washing, a constant pressure of about 225 g/cm² was applied to the tape, and the tape was then slowly removed at a constant speed to collect stratum corneum. This operation was repeated with 10 strips of tape. The stratum corneum collected from the surface with 1st and 2nd strips of tape (A group), with from 3rd to 5th strips of tape (B group), with from 6th to 8th strips of tape (C group), and with 9th and 10th strips of tape (D group) were pooled respectively, and libraries were prepared as in the method B of Example 1, sequencing was performed, and gene expression was detected. According to the report by Caroline Meyer Olesen, et al. (Scientific Reports, 9: 12217, 2019), stratum corneum tape can collect stratum corneum with a thickness of 1.3 **µ**m/strip up to the 5th strip and a thickness of 0.7 **µ**m/strip by the 6th to 10th strips. According to this report, it is inferred that stratum corneum is collected from the surface at a depth of from 0 to 2.6 **µ**m in the group A, from 2.7 to 6.6 **µ**m in the group B, from 6.7 to 8.8 **µ**m in the group C, and from 8.9 to 10.3 **µ**m in the group D.

Analysis of sequencing data detected 124 genes of which the expression level was increased 1.3 times or more in the group A compared to that in each of the groups B, C, and D (Table 3). A biological process (BP) search was performed for these genes by gene ontology (GO) enrichment analysis using public database Panther. As a result, it was demonstrated that stratum corneum in a shallow part collected by the 1st and 2nd strips of tape abundantly contains RNA involved in keratinization and lipid synthesis and metabolism (Table 4).

**[Table 3]**

| Highly expressed genes in group A (1st and 2nd strips of tape) | | | | |
|---|---|---|---|---|
| ABHD16B | DNAJB9 | KISS1R | PAPL | SLC5A1 |
| ADM | DSEL | KLK12 | PCTP | SMCR7 |
| AGAP2 | DUSP4 | KLK8 | PFKFB3 | SMPD3 |
| AIFM2 | DUX4L4 | KLK9 | PLCD3 | SPRR2F |
| ALDH1A3 | EHD1 | KRT34 | PLIN5 | SPRR2G |
| ANGPTL4 | FAM65C | KRTAP 1-5 | PNMA2 | SPRR3 |
| ANXA6 | FAR2 | KRTAP19-1 | PNPLA5 | STEAP4 |
| APOBEC3B | FBXO36 | KRTAP19-5 | PTGER4 | TADA2B |
| ARAP3 | FCHSD1 | KRTAP3-3 | RAI14 | TEAD4 |
| ASL | FICD | KRTAP5-5 | RAP1 GAP | TIGD2 |
| B3GNT5 | GADD45B | KRTAP7-1 | RDH10 | TIMP1 |
| BCAS4 | GBA | LCE3E | RELL2 | TINAGL1 |
| BEAN1 | GCC2 | LIPE | RGS10 | TMEM86A |
| BNIP3 | GFOD2 | LIPM | RRAD | TMPRSS11E |
| R3HCC1L | GLB1L2 | LNX1 | RUNDC3A | TNFSF9 |
| C19orf54 | GPLD1 | LOC100505839 | S100A7A | TOR2A |
| C1orf115 | HBEGF | LOC389791 | SCNN1G | ZDHHC23 |
| CARD9 | HEPHL1 | LOXL4 | SDR9C7 | ZG16B |
| CCL20 | HPSE | LPIN2 | SEC14L2 | ZNF432 |
| CHRNA9 | IL16 | LRRC15 | SEMA7A | ZNF75A |
| CNFN | IL1A | LYN | SERPINA9 | AZGP1P1 |
| CYP4F2 | IL1R2 | MFSD6L | SERPINB3 | GBAP1 |
| CYP4F3 | IL36G | MTRNR2L8 | SERPINB7 | LOC100170939 |
| DEFB4A | IL4R | NOMO3 | SLC26A9 | SERPINB4 |
| DEGS2 | INPP5D | OSBP2 | SLC39A2 | |

**[Table 4]**

| GO analysis of highly expressed 124 genes in group A (1st and 2nd strips of tape) | |
|---|---|
| GO biological process complete | FDR |
| keratinization (GO:0031424) | 2.16E-05 |
| keratinocyte differentiation (GO:0030216) | 7.94E-05 |
| skin development (GO:0043588) | 2.84E-04 |
| epidermal cell differentiation (GO:0009913) | 3.62E-04 |
| epidermis development (GO:0008544) | 4.80E-03 |
| lipid catabolic process (GO:0016042) | 6.32E-03 |
| cellular lipid catabolic process (GO:0044242) | 1.03E-02 |
| lipid metabolic process (GO:0006629) | 1.94E-02 |

## Claims

1. A method for preparing a nucleic acid sample derived from skin stratum corneum, the method comprising:
extracting nucleic acid from stratum corneum collected from a skin surface to prepare a water-soluble alcohol aqueous solution containing the nucleic acid, wherein the stratum corneum is stratum corneum within a depth of 5 **µ**m from the skin surface;
contacting the nucleic acid in the water-soluble alcohol aqueous solution with a solid-phase material to separate the nucleic acid, wherein a concentration of the water-soluble alcohol in the water-soluble alcohol aqueous solution is from 39 to 50 vol% before the contact with the solid-phase material; and
amplifying, or reverse-transcribing and amplifying, the separated nucleic acid in the presence of single-stranded nucleic acid-binding protein to prepare a nucleic acid sample, wherein the amplification is amplification by multiplex PCR, and the single-stranded nucleic acid-binding protein is T4GP32 or a variant thereof.

2. The method according to claim 1, further comprising collecting stratum corneum from the skin surface.

3. The method according to claim 1 or 2, wherein the stratum corneum collected from the skin surface is stratum corneum within four stratum corneum layers from the skin surface.

4. The method according to any one of claims 1 to 3, wherein the collection of stratum corneum from a skin surface is collection of stratum corneum by tape stripping.

5. The method according to claim 4, wherein adhesive tape used in the tape stripping is acrylic adhesive tape.

6. The method according to any one of claims 1 to 5, wherein the nucleic acid extracted from stratum corneum is RNA or DNA.

7. The method according to claim 6, wherein the nucleic acid extracted from stratum corneum and the separated nucleic acid are RNA, and the separated RNA is subjected to reverse transcription and amplification in the presence of the single-stranded nucleic acid-binding protein to prepare a nucleic acid sample.

8. The method according to claim 7, wherein the reverse transcription and amplification are multiplex RT-PCR, and an initial concentration of the single-stranded nucleic acid-binding protein in a reaction solution for the multiplex RT-PCR is from 3 to 300 **µ**g/mL.

9. The method according to claim 6, wherein the nucleic acid extracted from stratum corneum and the separated nucleic acid are DNA, and the separated DNA is amplified in the presence of the single-stranded nucleic acid-binding protein to prepare a nucleic acid sample.

10. The method according to claim 9, wherein an initial concentration of the single-stranded nucleic acid-binding protein in a reaction solution for amplification is from 3 to 300 **µ**g/mL.

11. The method according to any one of claims 1 to 10, wherein the water-soluble alcohol is at least one selected from the group consisting of methanol, ethanol, isopropanol, n-propanol, and butanol.

12. The method according to any one of claims 1 to 11, wherein the T4GP32 or a variant thereof is protein that consists of an amino acid sequence of SEQ ID NO: 1 or an amino acid sequence having a sequence identity of at least 90% with the sequence and binds to single-stranded nucleic acid.

13. The method according to any one of claims 1 to 12, wherein the solid-phase material is a silica-based solid-phase material.

14. A method for detecting gene expression in skin, the method comprising detecting gene expression in the nucleic acid sample derived from skin stratum corneum prepared by the method according to any one of claims 1 to 13.
